Europäisches Patentamt

(19) European Patent Office

Office européen des brevets

(11) Numéro de publication : **0 344 280 B1**

(12) **FASCICULE DE BREVET EUROPEEN**

(45) Date de publication du fascicule du brevet :
04.12.91 Bulletin 91/49

(51) Int. Cl.⁵ : **A61M 16/00**

(21) Numéro de dépôt : **89900612.6**

(22) Date de dépôt : **13.12.88**

(86) Numéro de dépôt international :
**PCT/FR88/00605**

(87) Numéro de publication internationale :
**WO 89/05669 29.06.89 Gazette 89/14**

(54) **Procédé de fonctionnement d'un dispositif de ventilation artificielle et un tel dispositif.**

(30) Priorité : **18.12.87 FR 8718327**

(43) Date de publication de la demande :
**06.12.89 Bulletin 89/49**

(45) Mention de la délivrance du brevet :
**04.12.91 Bulletin 91/49**

(84) Etats contractants désignés :
**AT BE CH DE GB IT LI LU NL SE**

(56) Documents cités :
**EP-A- 0 046 570**
**EP-A- 0 080 155**
**WO-A-87/02566**
**GB-A- 2 079 984**

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **CHOPIN, Claude**
**Route Cailles, Hameau Drumetz**
**F-59710 Attiches (FR)**
Inventeur : **CHAMBRIN, Marie-Christine**
**14, rue du Trianon**
**F-59139 Wattignies (FR)**
Inventeur : **BARANCOURT, Hervé**
**Domaine Universitaire**
**F-59650 Villeneuve-d'Ascq (FR)**
Inventeur : **FLOQUET, Nicolas**
**Domaine Universitaire**
**F-59650 Villeneuve-d'Ascq (FR)**

(74) Mandataire : **Lepage, Jean-Pierre**
**Cabinet Lepage & Aubertin Innovations et Prestations S.A. 23/25, rue Nicolas Leblanc**
**B.P. 1069**
**F-59011 Lille Cédex 1 (Nord) (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

**Description**

L'invention est relative à un procédé de fonctionnement d'un dispositif de ventilation artificielle ainsi qu'à un tel dispositif.

Elle trouvera notamment son application dans le domaine de la construction d'appareils médicaux et notamment d'appareils pour l'aide inspiratoire d'un patient.

Il est courant, dans le domaine médical, de faire appel à une technique que l'on appelle aide inspiratoire ou encore technique de ventilation artificielle, dans différents cas, notamment lorsqu'on est en présence de problèmes neurologiques, d'insuffisances respiratoires, voire même lors d'interventions chirurgicales et phases post-opératoires.

De telles techniques sont connues de longues dates et on recherche de plus en plus à diminuer l'agression barométrique pour le patient lorsqu'on lui procure une aide inspiratoire.

En effet, rappelons que, lorsqu'un individu a une ventilation naturelle spontanée, le sujet sain, dans son cycle respiratoire provoque l'inspiration par une dépression intérieure et une expiration par compression du volume d'air de ses voies respiratoires.

Au contraire, lorsqu'on est en ventilation artificielle pendant la phase d'inspiration, on insuffle vers le patient un mélange de fluide gazeux respirable; on se retrouve donc en pression lors de la phase d'inspiration et non pas en dépression comme naturellement lors de la ventilation spontanée.

Il est connu un dispositif d'aide respiratoire dans lequel la ventilation artificielle est contrôlée. Dans ce cas, la ventilation contrôlée joue le rôle d'une prothèse prenant en compte tout le travail ventilatoire du malade; c'est la ventilation contrôlée totale.

Depuis, d'autres techniques de ventilation, de plus en plus adaptées aux patients, ont vu le jour et permettent aux malades de prendre en compte progressivement une partie de son travail.

Un premier mode assisté de ventilation est connu, c'est le cas de la ventilation auto-déclenchée et de l'aide inspiratoire.

Dans ces modes assistés, la dépression provoquée par une inspiration du malade déclenche la phase inspiratoire, soit avec un volume et une durée fixés par la machine, soit avec le débit gazeux nécessaire pour maintenir une pression positive fixée par l'opérateur.

Ces modes de ventilation assistés présentent de nombreux inconvénients résidant d'une part dans le fait que le patient doit fournir un travail inspiratoire important selon la machine, et d'autre part dans les risques que provoquent une telle ventilation tels que des risques de tachypnée, d'alcalose ventilatoire, ou encore d'hyper-insufflation.

Plus récemment, d'autres types de ventilation artificielle fonctionnant selon des modes asservis ont été développés dans le but d'éviter un passage trop brutal de la ventilation contrôlée totale à la ventilation spontanée naturelle exclusive, de permettre une réadaptation progressive du patient et pour accroître sa sécurité. Parmi ceux-ci, on peut citer la ventilation contrôlée intermittente, la ventilation imposée variable, ou encore le mode de ventilation asservie au $CO_2$.

Dans un dispositif de ventilation contrôlée intermittente, celle-ci est imposée au malade et on force par exemple n-cycles contrôlés pour x-cycles spontanés.

Ce mode de ventilation présente des inconvénients qui résident dans la difficulté de réglage et dans la nécessité d'interventions manuelles pour régler le dispositif en fonction de l'évolution de la réanimation.

Dans le mode de ventilation imposée variable, tel que le décrit par exemple le document GB-A-2.079.984, celle-ci est asservie à une consigne de ventilation minute minimale, appelée ventilation minute de consigne ou de référence. Ainsi dans ce dispositif, on contrôle la différence entre, l'intégrale de la ventilation minute de référence et l'intégrale du débit instantané du patient, que l'on compare à une valeur de seuil de volume. Dès que ce seuil est atteint, l'unité de commande envoie au ventilateur un ordre pour délivrer au patient un volume courant de valeur prédéterminée et ce, jusqu'à ce que la consigne de ventilation minute ou valeur de référence soit respectée.

L'inconvénient majeur de ce type de ventilation est qu'il nécessite cinq réglages, dont deux habituellement utilisés pour la ventilation d'un patient à savoir la ventilation minute, minimale dans le cas décrit, et le volume courant imposé lorsque l'aide est nécessaire, et trois seuils dont la signification ne correpond pas à des paramètres habituels de la ventilation. Ces seuils, difficiles à évaluer pour le médecin, conditionnent le fonctionnement correct du système de commande.

Enfin, le dernier mode de ventilation asservie au $CO_2$ dans lequel c'est le $CO_2$ expiré maximum qui, surveillé et comparé à des seuils, commande le passage de la ventilation contrôlée à la ventilation spontanée ou inversement, présente des avantages par rapport aux dispositifs précités mais suppose un malade dont l'état cardio-respiratoire reste stable.

Cela étant, on connaît des documents EP-A-046.570 et WO-A-8.702.566 des systèmes de surveillance pour dispositifs de ventilation artificielle qui permettent essentiellement de visualiser ce qui se passe au niveau du ventilateur. Toutefois, il n'y a aucune coopération entre le système de surveillance et le ventilateur et aucune régulation de ce dernier par le dispositif de surveillance.

L'analyse du problème posé par l'assistance inspiratoire d'un patient montre qu'il est intéressant de limiter l'agressivité de la ventilation et d'autre part de piloter en permanence la ventilation artificielle pour tenir compte au maximum de la ventilation spontanée naturelle du patient afin que le malade prenne en charge une partie de son travail inspiratoire et ainsi facilite sa rééducation et/ou son sevrage de l'assistance ventilatoire.

les inconvénients des dispositifs d'aide inspiratoire connus à ce jour ne permettent pas de donner entière satisfaction aux réanimateurs dont l'intervention manuelle est souvent demandée car les dispositifs ne sont pas régulés correctement pour autoriser un ajustement de l'aide inspiratoire à l'effort que le malade doit faire et ne sont pas sans risque d'hypoventilation ou d'hyper-ventilation car le volume délivré lors de la phase d'insufflation n'est pas entièrement contrôlé.

le but de la présente invention est de proposer un procédé de régulation d'un dispositif de ventilation artificielle ainsi qu'un dispositif de ventilation artificielle pour l'aide inspiratoire d'un patient qui permettent de pallier les différents inconvénients précités des dispositifs connus.

Un des buts de la présente invention est de proposer un procédé de régulation d'un dispositif de ventilation artificielle qui permette au dispositif de ventilation le mettant en oeuvre de réaliser un mode de ventilation artificielle intermédiaire entre la ventilation contrôlée et la ventilation spontanée naturelle du patient. Ce mode intermédiaire est fonction des performances du malade et tient compte de la propre ventilation spontanée du patient.

Ainsi, ce mode permet de concevoir un système capable d'évoluer automatiquement entre les deux modes extrêmes de ventilation, c'est-à-dire qui évolue de lui-même de la ventilation spontanée à la ventilation contrôlée ou inversement.

Un autre but de la présente invention est de proposer un procédé de régulation d'un dispositif de ventilation artificielle et un dispositif pour l'aide inspiratoire d'un patient dans lequel le volume courant de fluide gazeux respirable insufflé dans le malade à chaque cycle est contrôlé.

Un autre but de la présente invention est de proposer un dispositif de ventilation artificielle pour l'aide inspiratoire d'un patient qui, notamment, grâce à son procédé de fonctionnement permet d'améliorer les caractéristiques de l'insufflation en délivrant au patient une aide inspiratoire quand l'effort demandé est le plus important c'est-à-dire en début d'inspiration.

Un autre but de la présente invention est de proposer un procédé de régulation d'un dispositif de ventilation artificielle ainsi d'un dispositif le mettant en oeuvre qui permettent de minimiser les interventions manuelles du réanimateur en le dispensant de toute obligation de réajustement selon l'évolution de la réanimation.

D'autres buts et avantages de la présente invention apparaîtront au cours de la description qui va suivre qui n'est donnée qu'à titre indicatif et qui n'a pas pour but de la limiter.

Selon l'invention, le procédé de fonctionnement d'un dispositif de ventilation artificielle pour l'aide inspiratoire volumétrique d'un patient, le dit patient présentant une propre ventilation spontanée naturelle définissant un volume courant patient, appelé "VT pat", ainsi qu'une pression des voies aériennes "Paw", le dit dispositif de ventilation artificielle, autorisant la génération d'un volume courant machine, appelé "VTmach", et autorisant une succession de cycles respiratoires en fonction des besoins du patient, de fréquence FR, chaque cycle respiratoire présentant une phase d'inspiration pendant laquelle on délivra au patient un volume de fluide gazeux respirable, appelé volume courant total "VTtot" égal à la somme du volume courant patient "VTpat" majoré du volume courant délivré par la machine "VTmach", à savoir "VTtot = VTpat + VTmach", ainsi qu'une phase d'expiration, procédé dans lequel :

    – on introduit une valeur de consigne de référence pour le dit dispositif, correspondant à une valeur de ventilation satisfaisante pour le patient en fonction de son état et de sa ventilation spontanée naturelle, appelée ventilation minute optimale: "Vmn.opt",

    – on contrôle le volume courant total délivré au patient à chaque cycle respiratoire: "VTtot", lequel procédé est caractérisé par le fait que :

    – on introduit une valeur de consigne de référence pour le dit dispositif, correspondant à une valeur minimale du volume courant "VT mini", avec "VTtot $\geq$ VT mini",

    – on introduit une valeur de consigne de référence pour le dit dispositif, correspondant à une valeur maximale de pression des voies aériennes pour le patient: "Paw max",

    – on asservit la ventilation artificielle à la consigne de ventilation minute "Vmn opt", de volume courant minimum "VT mini", et de pression des voies aériennes maximum "Paw max", de façon à satisfaire la relation "Vmn opt = FR x VT tot" pour faire évoluer automatiquement la ventilation artificielle de la ventilation spontanée à une ventilation contrôlée, ou inversement, en fonction de la ventilation spontanée naturelle du patient.

En outre, le dispositif de ventilation artificielle, pour l'aide inspiratoire volumétrique d'un patient, selon l'invention, le dit patient présentant une propre ventilation spontanée naturelle définissant un volume courant patient, appelé "VT pat", ainsi qu'une pression des voies aériennes "Paw", le dit dispositif de ventilation artificielle, autorisant la génération d'un volume courant machine, appelé "VTmach", et autorisant une succession de cycles respiratoires en fonction des besoins du patient, de fréquence "FR", chaque cycle respiratoire présentant une phase d'inspiration pendant laquelle on délivra au patient un volume de fluide gazeux respirable, appelé volume courant total "VTtot" égal à la somme du volume courant patient "VTpat" majoré du volume courant délivré par la machine "VTmach", à savoir "VTtot = VTpat + VTmach", ainsi qu'une phase d'expiration, le dit dispositif comprenant :

    – une source d'alimentation en fluide gazeux respirable, permettant au moins une alimentation en fluide sous pression lors de la ventilation artificielle,

    – des moyens de canalisation du dit fluide gazeux vers le patient,

    – des moyens de contrôle du volume courant total "VTtot" délivré au patient à chaque cycle respiratoire,

    – des moyens d'introduction de valeurs de consigne de référence pour le dit dispositif, correspondant au moins à une valeur de ventilation satisfaisante pour le patient en fonction de son état et de sa ventilation spontanée naturelle, appelée ventilation minute optimale: "Vmn.opt", lequel dispositif est caractérisé par le fait que :

    – les dits moyens d'introduction de valeurs de consigne de référence permettent l'introduction de :

    . une valeur de consigne de référence pour le dit dispositif, correspondant à une valeur minimale du volume courant que doit inspirer le patient, appelée volume courant mini: "VT mini", avec "VTtot ≧ VTmini",

    . une valeur de consigne de référence pour le dit dispositif, correspondant à une valeur maximale de pression des voies aériennes pour le patient: "Paw max", et qu'il comporte :

    – des moyens d'asservissement de la ventilation artificielle vérifiant à chaque cycle la relation : "Vmn opt = FR x VT tot" agissant sur l'alimentation en fluide de la ventilation artificielle sous pression selon l'inspiration naturelle spontanée du patient pour faire évoluer automatiquement la ventilation artificielle entre la ventilation spontanée et une ventilation contrôlée.

La présente invention sera mieux comprise à la lecture de la description suivante accompagnée des dessins en annexe qui en font partie intégrante.

La figure 1 illustre schématiquement un mode de réalisation du dispositif de ventilation artificielle pour l'aide inspiratoire volumétrique d'un patient selon la présente invention.

La figure 2 montre le graphe du débit de fluide gazeux respirable délivré au patient par le dispositif de ventilation artificielle de la présente invention en fonction du temps.

Les figures 3a à 3c montrent le schéma des phases des différents organes de commande actionneurs du dispositif de ventilation artificielle de la figure 1 permettant notamment l'obtention du graphe tel que représenté à la figure 2.

Les figures 4a à 4b illustrent le principe physique de l'aide inspiratoire délivré par le dispositif de la présente invention et fournissant une aide maximale au début de l'insufflation.

Les figures 5a à 5g illustrent schématiquement l'organigramme de fonctionnement d'un dispositif de ventilation artificielle selon la présente invention.

L'invention vise un procédé de fonctionnement d'un dispositif de ventilation artificielle ainsi qu'un dispositif de ventilation utilisant notamment le dit procédé trouvant particulièrement son application pour l'aide inspiratoire volumétrique d'un patient.

D'une manière générale, un dispositif de ventilation artificielle autorise une succession de cycles respiratoires, de fréquences FR, chaque cycle présentant une phase d'inspiration lors de laquelle on délivre, pendant un temps d'insufflation TI, au patient un volume de fluide gazeux respirable, appelé volume courant total VTtot, ainsi qu'une phase d'expiration définie par un temps d'expiration TE.

Pendant cette aide inspiratoire, il est à noter que le dit patient présente sa propre ventilation spontanée naturelle, VTpat, et un des buts de la présente invention est d'autoriser un fonctionnement du dispositif de ventilation artificielle par lequel on fournit une aide inspiratoire volumétrique en fonction des besoins du patient.

Le volume courant total VTtot est la somme du volume courant résultant de la ventilation spontanée du patient VTpat et du volume courant délivré par la "machine", c'est-à-dire par le dispositif de ventilation artificielle, appelé VTmach.

Pour ce, selon le procédé de fonctionnement du dispositif de ventilation artificielle de la présente invention, on réalise la succession des étapes suivantes qui rendent originale l'invention par rapport à l'état de la technique connu.

Tout d'abord, on fixe une valeur théorique de ventilation satisfaisante pour le patient en fonction de son état et de sa ventilation spontanée naturelle. Cette valeur théorique est appelée ventilation minute optimale Vmn opt. et est décidée par le réanimateur qui fixe cette valeur selon les besoins du malade.

Ensuite, on fixe une valeur minimale du volume courant que doit inspirer le patient à chaque cycle respiratoire. Cette valeur est appelée volume courant minimal VT mini et est également décidée par le réanimateur afin qu'il y ait des échanges gazeux satisfaisants lors de la respiration.

Ensuite, on contrôle le volume courant total VTtot délivré au patient à chaque cycle respiratoire par l'intermédiaire de tout moyen de détection approprié, avec VTtot ≧ VTmini.

A des fins de sécurité du procédé de fonctionnement de l'invention, on fixe une valeur maximale de pression des voies aériennes Paw max, lors de la phase d'inspiration pour limiter la pression d'insufflation à une valeur maximale admissible pour le patient.

Enfin, on asservit la ventilation artificielle à la consigne de ventilation minute optimale Vmn opt et de volume courant minimum VT mini.

La relation $Vmn_{opt} = FR \times VT_{tot}$ peut être satisfaite pour différentes valeurs de fréquence respiratoire FR et de $VT_{tot}$ tant que $VT_{tot}$ est supérieure à VT mini, FR supérieure à la fréquence de sécurité et la pression des voies aériennes inférieure à la valeur maximum.

Le système évolue automatiquement de la ventilation spontanée à une ventilation contrôlée ou inversement, en fonction de la ventilation spontanée et naturelle du patient.

Selon une autre caractéristique de la présente invention, il est à noter que le dispositif de ventilation artificielle est prévu tel pour que celle-ci, lors de l'insufflation pendant la phase d'inspiration, délivre une aide maximale en début d'inspiration c'est-à-dire à petit volume pulmonaire. En effet, rappelons que c'est au début de l'inspiration que l'individu doit fournir un effort maximum pour "ouvrir" ses alvéoles pulmonaires, cet effort étant ensuite beaucoup plus faible pour les maintenir ouvertes.

Le procédé de régulation du dispositif de ventilation artificielle de la présente invention sera décrit plus en détail dans la suite de la présente demande et, pour faciliter sa compréhension, nous décrirons d'abord un mode de réalisation d'un dispositif de ventilation artificielle selon la présente invention.

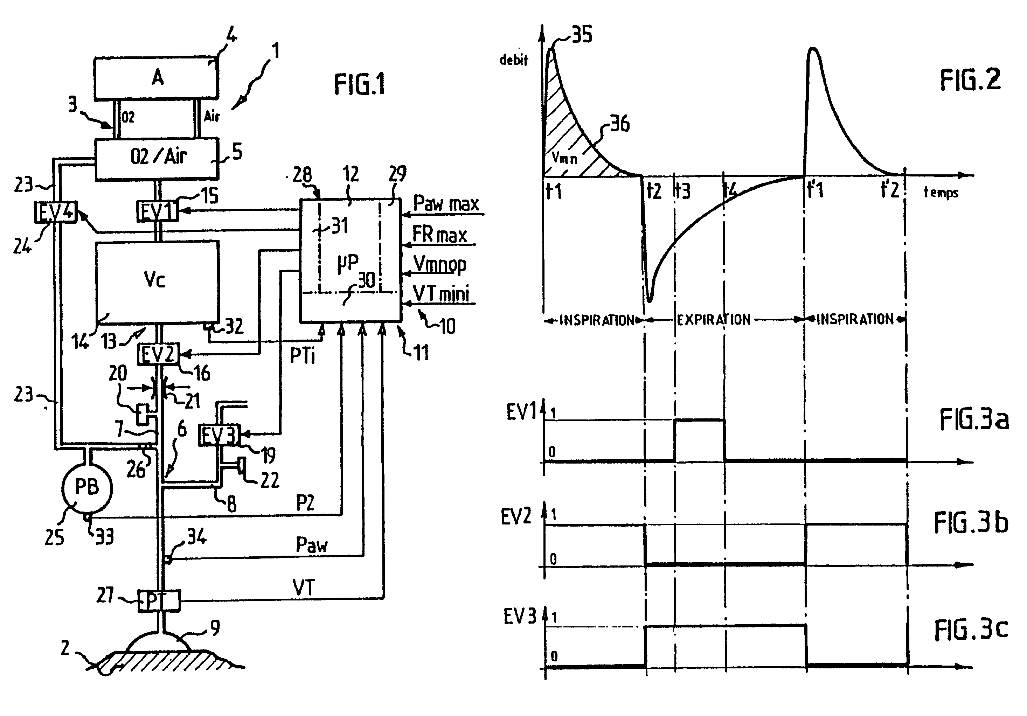

La figure 1 montre une réalisation d'un dispositif de ventilation artificielle 1, selon la présente invention, qui trouvera notamment son application pour l'aide inspiratoire volumétrique d'un patient 2.

Ce dispositif comprend au moins une source d'alimentation 3 en fluide gazeux respirable permettant au moins une alimentation en fluide sous pression lors de la ventilation artificielle.

Cette source d'alimentation 3 est notamment constituée par une alimentation 4, qui peut autoriser, par exemple, une pression jusque de l'ordre de 3 bars, et d'un groupe mélangeur 5 oxygène-air qui permet de réaliser un fluide gazeux respirable de concentration ajustable.

Par ailleurs, le dispositif comporte également des moyens de canalisations 6 du dit fluide gazeux vers le patient, notamment constitués d'une tubulure d'insufflation 7 et d'une tubulure d'expiration 8, ces moyens étant réalisés selon des techniques traditionnelles connues de l'Homme du Métier. Au niveau du patient 2, l'arrivée à la bouche se fait par l'intermédiaire d'un T 9 ou tout autre dispositif de masque adapté.

Selon une caractéristique de la présente invention, le dispositif de ventilation artificielle 1 comprend en combinaison :
— des moyens 10 d'introduction de consignes de référence, au moins aptes à fixer une valeur théorique de ventilation optimale $Vmn_{opt}$ pour le patient, une valeur minimale de volume courant VT mini et une valeur maximale de pression des voies aériennes Paw max,
— des moyens 11 de contrôle du volume courant total $VT_{tot}$ délivré au patient à chaque cycle respiratoire,
— des moyens 12 d'asservissement de la ventilation artificielle vérifiant à chaque cycle la relation $Vmn_{opt} = FR \times VT_{tot}$ agissant sur l'alimentation en fluide sous pression de la ventilation artificielle selon l'inspiration naturelle spontanée du patient, pour faire évoluer automatiquement la ventilation artificielle entre la ventilation spontanée et une ventilation contrôlée.

Par ailleurs, le dispositif de ventilation artificielle 1 de la présente invention, selon une autre caractéristique importante de la présente invention, comprend des moyens 13 pour délivrer au patient une aide maximale en début d'inspiration, c'est-à-dire à petit volume pulmonaire, pendant la phase d'inspiration lors de l'insufflation par le dit dispositif 1.

Plus précisément, les dits moyens 13 précités comprennent une enceinte réservoir 14 dont le volume VC est déterminé, intercalé sur la dite canalisation d'inspiration 7 entre la dite alimentation sous pression 3 et le patient 2 tel que le montre la figure 1.

Par ailleurs, cette enceinte 14 est prévue pour résister à la pression car dans le fonctionnement du dispositif 1, la dite enceinte 14 peut être remplie en fluide gazeux sous pression à chaque cycle respiratoire à une pression de travail $PT_1$ prédéterminée.

Ceci sera notamment autorisé grâce à une électrovanne EV1 repérée 15 intercalée en série sur la tubulure reliant le mélangeur 5 et l'enceinte 14.

En outre, les dits moyens 13 comportent des moyens de décharge 16 du dit volume VC à pression $PT_1$ placés en aval de la dite enceinte 14 pour notamment la vider brutalement.

Ces moyens de décharge sont substantiellement constitués par une électrovanne EV2 repérée 16 intercalée en série dans la canalisation d'inspiration 7 qui, rappelons-le, relie le patient 2 à l'enceinte 14.

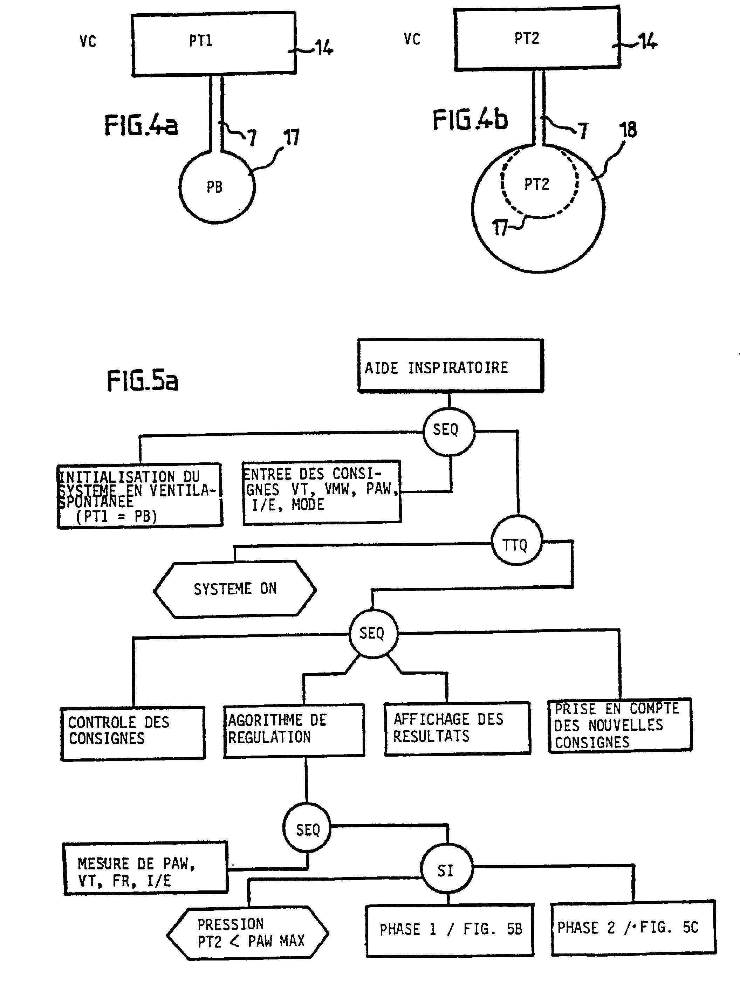

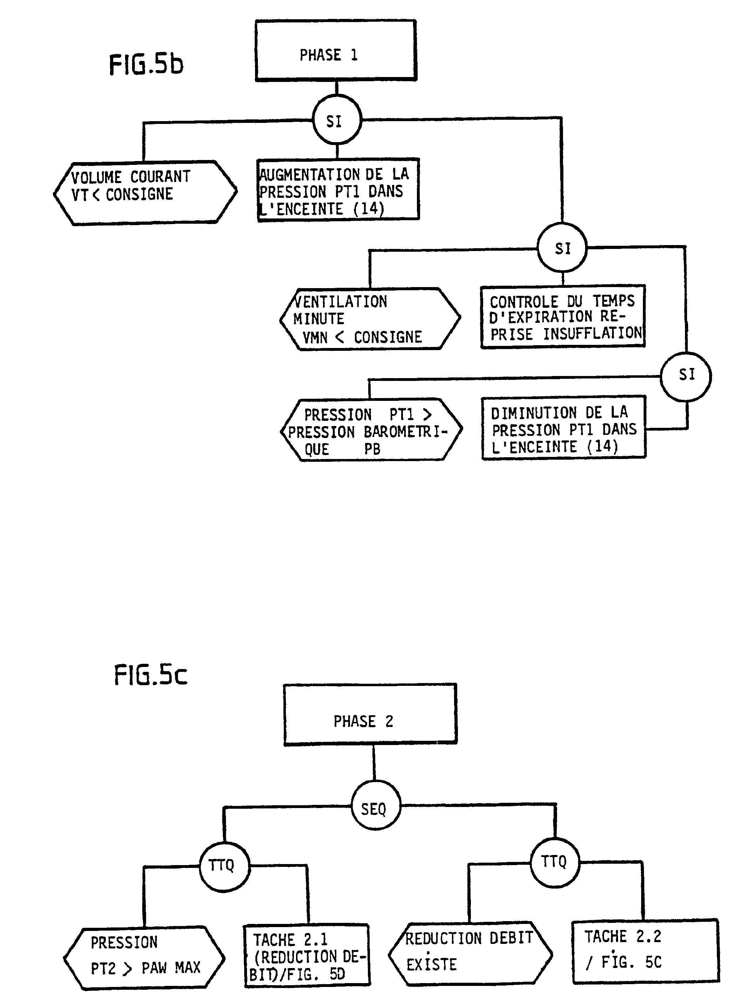

Les figures 4a et 4b montrent le principe de fonctionnement des moyens 13 qui permettent l'aide inspiratoire maximale au début de l'insufflation c'est-à-dire à petit volume pulmonaire.

Plus précisément, la figure 4a schématise en 14 la dite enceinte réservoir, en 17 le volume pulmonaire du patient en fin d'expiration, et en 7 la canalisation d'insufflation.

En fin d'expiration, on suppose que l'enceinte réservoir 14 de volume VC est remplie à une pression de travail $PT_1$ fonction du volume à délivrer au patient.

Tant que l'électrovanne EV2 n'est pas ouverte, le fluide gazeux sous pression $PT_1$ dans l'enceinte 14 est conservé étant donné que celle-ci n'est pas reliée directement au patient.

En effet, au niveau des poumons, on se trouve à la pression barométrique PB, le volume pulmonaire, schématisé et repéré 17, est égal à la valeur de la capacité résiduelle fonctionnelle du patient CRF.

La figure 4b montre la phase de décharge du fluide gazeux sous pression contenu dans l'enceinte 14. En début d'insufflation, on alimente l'électrovanne EV2 qui relie en communication l'enceinte 14 et le volume pulmonaire du patient. Ainsi, le dispositif délivre au patient le dit volume courant VTmach.

En fin d'insufflation, il existe une pression résultante $PT_2$ à l'intérieur du système enceinte 14 et poumons du malade, le volume pulmonaire correspondant à CRF + VT, schématisé en 18 sur la figure, VT représentant le volume courant insufflé au patient.

Etant donné la structure des éléments qui viennent d'être décrits et grâce à la décharge brutale du dit volume VT mach vers les poumons du patient, on obtient un pic de débit du fluide au début de l'insufflation tel que cela est illustré à la figure 2.

Plus précisément, sur cette figure, on représente le débit insufflé au patient par la ventilation artificielle en fonction du temps. A l'instant t1 correspond le début de la phase d'inspiration c'est-à-dire de l'insufflation et à l'instant t2 correspond la fin de la phase d'inspiration.

Etant donné la loi physique de la décharge, on obtient une courbe avec un pic 35 très rapproché de l'instant t1 qui correspond à la détente puis une décharge exponentielle 36 qui correspond à l'établissement du régime d'équilibre jusqu'en fin d'inspiration de l'instant t2.

Par ailleurs, le dispositif de la présente invention comporte également sur sa canalisation d'expiration 8 une électrovanne EV3, repérée 19 à la figure 1, qui permet le bon fonctionnement de la phase d'inspiration et autorise également l'expiration.

Les figures 3a à 3c montrent les états des trois électrovannes EV1 à EV3 durant les phases successives d'inspiration et d'expiration.

L'inspiration s'effectue entre le temps repéré t1 et t2 et l'expiration durant le temps repéré t2 à t'1. Par ailleurs, par 0, on représente l'électrovanne fermée c'est-à-dire canalisation obturée et par 1, on représente l'électrovanne ouverte c'est-à-dire libre communication entre les éléments.

La séquence de fonctionnement est la suivante :
– l'électrovanne EV1 n'est ouverte durant l'expiration que le temps de remplir le caisson à la pression PT1, c'est-à-dire entre les instants t3 et t4,
– l'électrovanne EV2 est fermée durant toute l'expiration et ouverte durant toute l'insufflation,
– l'électrovanne EV3 par contre est fermée durant toute l'inspiration et ouverte durant toute l'expiration.

Selon une caractéristique du procédé de régulation du dispositif de la présente invention, on commande le découpage du cycle en phase inspiratoire et en phase expiratoire en détectant les instants de débit nul en fin d'inspiration et en fin d'expiration du patient. En ce qui concerne la séquence de fonctionnement des électrovannes et du système de décharge du volume VC, le signal d'ouverture et de fermeture des électrovannes EV2 et EV3 est donné par les instants de débit nul autant t1, t2, t'1, etc.....

La régulation du dispositif de l'invention tient compte de ces instants de temps et en particulier :
– la durée (t1 - t2) est fonction des temps de passage par les points de débit nul,
– la durée (t2 - t3) est fixée et constante,
– la durée (t3 - t4) est variable en fonction de la pression de travail $PT_1$ à atteindre.

En complément des électrovannes précitées, la canalisation d'inspiration 7 comprend également une valve de surpression 20 ainsi que des moyens 21 de réduction du débit, et la canalisation d'expiration 8 comporte également une valve de surpression 22.

Par ailleurs, le dispositif comporte également une alimentation en fluide gazeux respirable sensiblement à pression barométrique apte à alimenter le patient pour sa ventilation naturelle spontanée.

Cette dite alimentation peut être réalisée par une simple mise à l'air de la canalisation d'inspiration 7 au-travers d'une valve d'air additionnelle anti-retour.

Toutefois, selon un mode de réalisation avantageux, tel qu'illustré à la figure 1, cette alimentation en fluide gazeux respirable à pression barométrique utilise de l'air mélangé avec de l'oxygène et profite de la présence du mélangeur 5 du dispositif. Ainsi, on dérive le mélange au moyen d'une canalisation 23 sur laquelle est intercalée une électrovanne EV4 repérée 24, pour alimenter par exemple un ballon réservoir 25 à pression sensiblement voisine de celle de la pression barométrique.

Ce ballon 25 est ensuite connecté sur la canalisation d'inspiration 7 par l'intermédiaire d'une valve anti-retour 26 de technique et de fonctionnement connus par l'Homme du Métier.

Ainsi, lorsque l'électrovanne EV4 est ouverte, on remplira le ballon 25 à une pression $P_2$ légèrement supérieure à la Pression Barométrique PB par un flux d'air enrichi d'oxygène qui sera utilisé lors de la phase d'inspiration en ventilation spontanée naturelle du patient. Par contre, lorsque la ventilation artificielle sera en service, la valve 26 fera anti-retour pour éviter que la décharge du volume VC de l'enceinte 14 ne se fasse par la canalisation 23 et que toute la quantité de fluide déchargé soit dirigée vers le patient 2.

Par ailleurs, un tel montage permet, lorsque la ventilation naturelle spontanée du patient n'est pas suffisante de la compléter par la ventilation artificielle dans un mode dit intermédiaire dont l'asservissement va être décrit plus en détail ultérieurement.

En ce qui concerne la structure du dispositif, il est à noter la présence de moyens 27 de mesure du débit du fluide inspiré et/ou expiré par le patient placé juste en amont du patient 2. .

Ces moyens sont avantageusement constitués par un capteur de débit de type pneumotachographe. A ce niveau, il est également à remarquer qu'une intégration du signal de débit mesuré permettra de connaitre le volume inspiré ou expiré du patient.

A cet égard, le dispositif de la présente invention comprend des moyens de traitement du signal de débit ainsi mesurés pour d'une part détecter les instants de débit nul en fin d'inspiration et/ou d'expiration et d'autre part déterminer en fonction du débit mesuré le volume courant total VT inspiré par le patient.

Par ailleurs, des capteurs de pression 32 à 34 sont prévus pour mesurer respectivement notamment la pression de travail dans l'enceinte 14, la pression dans le ballon 25, la pression des voies aériennes Paw, comme cela est illustré à la figure 1.

Enfin, pour autoriser l'asservissement du procédé de régulation de la présente invention, le dispositif de ventilation artificielle comprend des moyens d'incrémentation et/ou de décrémentation de la ventilation artificielle agissant sur l'alimentation en fluide sous pression permettant d'arriver à ce que la relation Vmn = FR x VT soit satisfaite en fonction de la propre alimentation spontanée naturelle du patient en fluide gazeux respirable en respectant les dites consignes de référence.

A cet égard, le dispositif comporte des moyens de calcul et de commande 28 substantiellement constitués par :

– une unité centrale à microprocesseur permettant le déroulement séquentiel des différentes étapes,
– une interface d'entrée 29, 30 recueillant les valeurs de référence des consignes telles que ventilation minute optimale, volume courant mini, pression maximum des voies aériennes, fréquence respiratoire maximale, et les informations mesures telles que pression de travail dans l'enceinte PTi, pression des voies aériennes Paw, débit d'inspiration et/ou d'expiration, et éventuellement pression de travail dans le ballon 25,
– une interface de sortie 31 contrôlant la commande séquentielle des électrovannes 15, 16, 19, 24 pour autoriser soit une ventilation spontanée, soit une ventilation contrôlée totale, soit une ventilation artificielle intermédiaire, et/ou la phase d'expiration.

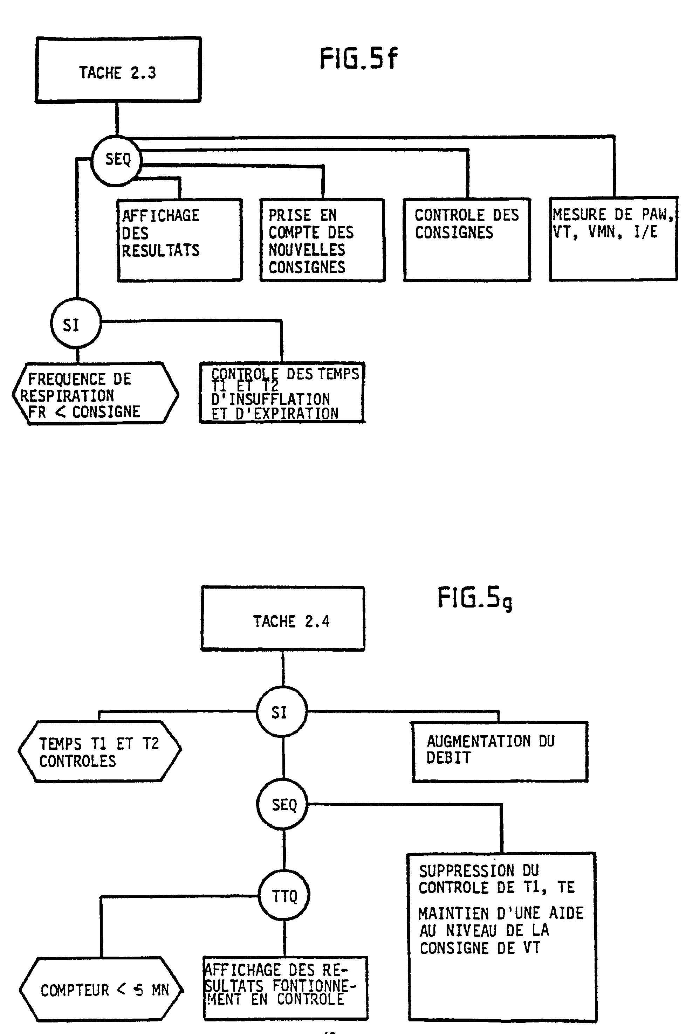

En ce qui concerne le procédé de régulation du dispositif de la présente invention, les figures 5a à 5g montrent l'organigramme qui schématise le principe de l'asservissement.

Plus précisément, la figure 5a montre l'organigramme de base et les phases 1 et 2 sont respectivement illustrées sur les figures 5b et 5c.

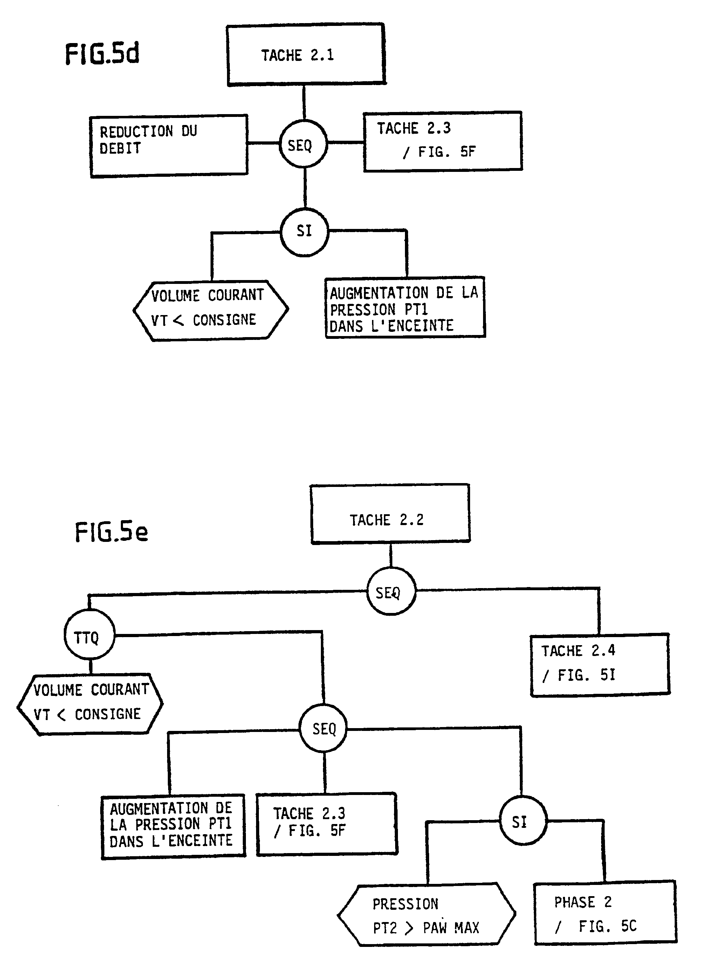

De même, en ce qui concerne la figure 5c, les taches 2.1 et 2.2 sont respectivement illustrées sur les figures 5d et 5e. Les figures 5f et 5g montrent respectivement les taches 2.3 et taches 2.4 dont il est question aux figures 5d et 5e.

L'écriture de ces organigrammes ne nécessite pas de commentaires supplémentaires car ceux-ci s'explicitent d'eux-mêmes; toutefois, il est à noter que par le terme "seq", on signifie : "on réalise séquentiellement", par le terme "ttq", on signifie "tant que" et l'expression "si" est comprise comme une "condition" vérifiée ou non.

Par ailleurs, il est à noter que l'initialisation du système se fait en ventilation spontanée, les consignes de ventilation minute Vmn, de volume courant minimum VT, et de pression aérienne Paw maximale étant fixées.

Le calcul du volume courant VT, et de la fréquence FR est réalisé sur trois cycles.

Le test des consignes s'effectue notamment de la façon suivante : au cours de l'insufflation, la valeur de la pression aérienne Paw est constamment mesurée car il s'agit là d'une consigne de sécurité.

Si la valeur maximale Paw maxi est atteinte, on provoque alors la fermeture de l'électrovanne de décharge EV2, et la pression de remplissage de l'enceinte 14 n'augmente plus non plus.

Toutefois, il est à noter qu'une procédure d'asservissement du débit peut être envisagée à ce niveau, procédure incluse dans le diagramme représenté à la figure 5d.

Si le patient obéit lui-même à la consigne et satisfait la dite relation Vmn = FR x VT, l'effet de la ventilation artificielle est annihilé, sinon on incrémente la ventilation artificielle à chaque cycle jusqu'au moment où le patient obéit à la consigne avec l'aide inspiratoire.

Ainsi, le volume courant VT insufflé au patient est comparé à la consigne préalablement fixée. Si cette consigne n'est pas atteinte, on augmente alors la pression de travail PT1 dans l'enceinte 14. Cette incrémentation se fait avec un pas variable en fonction de l'écart entre la valeur mesurée et la valeur de consigne à atteindre.

Si la consigne de volume courant VT est respectée, on teste la valeur de la ventilation minute Vmn. Dans le cas où elle n'est pas atteinte, c'est la fréquence respiratoire du patient qui est trop basse, alors la durée de l'expiration est contrôlée et l'inspiration déclenchée de façon à respecter la valeur de la fréquence minimale FR mini.

Par contre, dans le cas où cette consigne est dépassée, et qu'il existe une aide inspiratoire, cette dernière est diminuée.

En effet, lorsque la consigne est atteinte avec l'aide inspiratoire, on décrémente progressivement la ventilation artificielle tout en respectant l'asservissement jusqu'au moment où le patient retrouve son mode de ventilation spontanée naturelle.

Par ailleurs, pour délivrer une aide maximale en début d'inspiration, c'est-à-dire à petit volume pulmonaire du patient, en ventilation artificielle, selon le procédé de régulation de la présente invention, à chaque phase d'inspiration, lorsque la ventilation artificielle est opérante, on insuffle au patient la décharge d'un volume VC déterminé de fluide gazeux respirable préalablement porté à une pression de travail préétablie PT1.

En effet, comme il a été décrit précédemment, le volume VC à pression PT1 est obtenu grâce à la présence de l'enceinte réservoir 14 dont l'entrée est commandée par l'électrovanne EV1 et dont la décharge est commandée par l'électrovanne EV2.

A titre d'exemple, avec un dispositif de ventilation artificielle aux caractéristiques suivantes :

- volume VC de l'enceinte 14 : 2 litres,
- alimentation en air comprimé jusque 3 bars,
- volume courant insufflé : de 0 à 1,5 litres avec un pas de 50 millilitres,
- pression d'insufflation comprise entre 0 et 120 $cmH_2O$,
- débit maximum d'insufflation atteint : 150 litres/mn,
- température du mélange gazeux respirable insufflé : 37°C,
- pression PTi dans l'enceinte 14 mesurée par un capteur de pression relative 0-5 bars d'une précision de 0,5 %,
- pressions Paw dans la canalisation d'inspiration et $P_2$ dans le ballon 25 mesurées par des capteurs de pressions différentielles,
- un capteur de débit du type pneumotachographe au niveau de la bouche du patient permettant une mesure dans les deux sens expiration et inspiration,

on a obtenu les performances maximales de :

- fréquence de respiration correspondante : 30 cycles/mn avec un rapport d'inspiration-expiration (I/E) de 0,2 , un temps inspiratoire de 0,4 seconde avec un volume d'un litre.

le bon fonctionnement du système est lié au bon fonctionnement des capteurs et la sécurité du système est assurée de la façon suivante :

- le fonctionnement du capteur de pression 32 mesurant la pression de l'enceinte réservoir 14 durant la phase de remplissage est contrôlé par le système. Si le signal qu'il délivre n'est pas croissant, on ferme alors l'électrovanne EV1 15 et une alarme est déclenchée.

Au niveau du capteur de pression différentielle 34 permettant la mesure de la pression des voies aériennes, une simple alarme est déclenchée en cas de variation nulle du signal car un tel défaut n'entraine pas une perturbation majeure du fonctionnement du système.

Pour ce qui est du capteur de débit 27, une panne de ce dernier entraine une ventilation minimale sur la base de la sécurité à l'apnée. De plus, si aucune inspiration n'est détectée au bout de dix secondes, une insufflation est déclenchée avec une consigne de remplissage de l'enceinte réservoir 14 préfixée.

Cela étant, pour prévenir les problèmes liés aux phénomènes de fuite ou de débranchement, un tableau de correspondance entre le volume insufflé et la pression de remplissage théorique est élaboré. En outre, on mesure l'écart $\Delta P$ entre la pression de remplissage et la pression théorique.

Si un débranchement, volontaire ou accidentel, survient avant le débitmètre 27, dans le cas où le patient dispose d'une ventilation spontanée naturelle faible, il se produirait une augmentation de la pression de remplissage du caisson de façon à augmenter le volume courant. On limite volontairement cette augmentation par la mesure de l'écart $\Delta P$.

Si $\Delta P$ correspond à une variation de la pression de remplissage supérieur de 10 % à la valeur théorique, cette dernière sert de consigne. De ce fait, on exclut tout risque d'hyper-insufflation liée à l'augmentation de la pression de remplissage. De plus, lorsque celle-ci est détectée, on déclenche une alarme.

D'autres problèmes peuvent également se produire en cas de fuites dans les moyens de canalisation ou autres.

Pour ce, une fuite en amont sera compensée au maximum par une augmentation de la pression de remplissage suffisante pour fournir le volume courant minimum.

Par contre, une fuite en aval du débitmètre sera détectée par la comparaison des volumes insufflés et expirés.

Enfin, le système informatique de calcul et d'asservissement sera contrôlé et en cas de panne de ce dernier, on déclenchera une alarme et le système restera ouvert en ventilation spontanée.

Naturellement, d'autres mises en oeuvre de la présente invention, à la portée de l'Homme de l'Art, auraient pu être envisagées sans pour autant sortir du cadre de celle-ci, telle que définie dans les revendications suivantes.

## Revendications

1. Procédé de fonctionnement d'un dispositif de ventilation artificielle pour l'aide inspiratoire volumétrique d'un patient (2), le dit patient (2) présentant une propre ventilation spontanée naturelle définissant un volume courant patient, appelé "VT pat", ainsi qu'une pression des voies aériennes "Paw", le dit dispositif de ventilation artificielle (1), autorisant la génération d'un volume courant machine, appelé "VTmach", et autorisant une succession de cycles respiratoires en fonction des besoins du patient, de fréquence "FR", chaque cycle respiratoire présentant une phase d'inspiration pendant laquelle on délivra au patient un volume de fluide gazeux respirable, appelé volume courant total "VTtot" égal à la somme du volume courant patient "VTpat" majoré du volume courant délivré par la machine "VTmach", à savoir "VTtot = VTpat + VTmach", ainsi qu'une phase d'expiration, procédé dans lequel :

– on introduit une valeur de consigne de référence pour le dit dispositif, correspondant à une valeur de ventilation satisfaisante pour le patient en fonction de son état et de sa ventilation spontanée naturelle, appelée ventilation minute optimale: "Vmn.opt",

– on contrôle le volume courant total délivré au patient à chaque cycle respiratoire: "VTtot", procédé caractérisé par le fait que :

– on introduit une valeur de consigne de référence pour le dit dispositif, correspondant à une valeur minimale du volume courant "VT mini", avec "VTtot ≥ VT mini",

– on introduit une valeur de consigne de référence pour le dit dispositif, correspondant à une valeur maximale de pression des voies aériennes pour le patient: "Paw max",

– on asservit la ventilation artificielle à la consigne de ventilation minute "Vmn opt", de volume courant minimum "VT mini", et de pression des voies aériennes maximum "Paw max", de façon à satisfaire la relation "Vmn opt = FR x VT tot" pour faire évoluer automatiquement la ventilation artificielle de la ventilation spontanée à une ventilation contrôlée, ou inversement, en fonction de la ventilation spontanée naturelle du patient.

2. Procédé de fonctionnement d'un dispositif de ventilation artificielle selon la revendication 1, caractérisé par le fait que la ventilation artificielle, lors de l'insufflation pendant la phase d'inspiration délivre une aide maximale en début d'inspiration à petit volume pulmonaire.

3. Procédé de fonctionnement d'un dispositif de ventilation artificielle selon la revendication 1, caractérisé par le fait que l'on contrôle le volume courant total "VTtot" pendant la phase d'inspiration et d'expiration et que l'on commande le découpage du cycle en phases inspiratoire et expiratoire en détectant les instants de débit nul en fin d'inspiration et en fin d'expiration du patient.

4. Procédé de fonctionnement d'un dispositif de ventilation artificielle selon la revendication 1, caractérisé par le fait que si le patient obéit de lui même à la consigne et satisfait la relation "Vmn opt = FR x VTtot", l'effet de la ventilation artificielle est annihilé, sinon on incrémente la ventilation artificielle à chaque cycle jusqu'au moment où le patient obéit à la consigne avec l'aide inspiratoire.

5. Procédé de fonctionnement d'un dispositif de ventilation artificielle selon la revendication 4, caractérisé par le fait que lorsque la consigne est atteinte avec l'aide inspiratoire, on décrémente progressivement la ventilation artificielle tout en respectant l'asservissement jusqu'au moment où le patient retrouve son mode de ventilation spontanée naturelle.

6. Procédé de fontionnement d'un dispositif de ventilation artificielle selon la revendication 2, caractérisé par le fait qu'à chaque phase d'inspiration, lorsque la ventilation artificielle est opérante, on insuffle au patient la décharge d'un volume déterminé de fluide gazeux respirable préalablement porté à une pression de travail préétablie.

7. Procédé de fontionnement d'un dispositif de ventilation artificielle selon la revendication 1, caractérisé par le fait que l'on fixe une valeur maximale de fréquence respiratoire FR pour prévenir le risque d'hyperventilation.

8. Dispositif de ventilation artificielle (1), pour la mise en oeuvre du procédé de fonctionnement selon la revendication 1, le dit dispositif comprenant :

– une source d'alimentation (3) en fluide gazeux respirable, permettant au moins une alimentation en fluide sous pression lors de la ventilation artificielle,

– des moyens de canalisation (6) du dit fluide gazeux vers le patient,

– des moyens (11) de contrôle du volume courant total "VTtot" délivré au patient à chaque cycle respiratoire,

– des moyens (10) d'introduction de valeurs de consigne de référence pour le dit dispositif, correspondant au moins à une valeur de ventilation satisfaisante pour le patient en fonction de son état et de sa ventilation spontanée naturelle, appelée ventilation minute optimale: "Vmn.opt", dispositif caractérisé par le fait que :

– les dits moyens (10) d'introduction de valeurs de consigne de référence permettent l'introduction de :

. une valeur de consigne de référence pour le dit dispositif, correspondant à une valeur minimale du volume courant que doit inspirer le patient, appelée volume courant mini: "VT mini", avec "VTtot ≥ VT mini",

. une valeur de consigne de référence pour le dit dispositif, correspondant à une valeur maximale de pression des voies aériennes pour le patient: "Paw max", et qu'il comporte :

– des moyens d'asservissement (12) de la ventilation artificielle vérifiant à chaque cycle la relation : "Vmn opt = FR x VT tot" agissant sur l'alimentation en fluide de la ventilation artificielle sous pression selon l'inspiration naturelle spontanée du patient pour faire évoluer automatiquement la ventilation artificielle entre la ventilation spontanée et une ventilation contrôlée.

9. Dispositif de ventilation artificielle selon la revendication 8, caractérisé par le fait qu'il comprend des moyens (13) pour délivrer au patient une aide maximale en début d'inspiration, c'est-à-dire à petit volume pulmonaire, pendant la phase d'inspiration lors de l'insufflation par le dit dispositif de ventilation artificielle.

10. Dispositif de ventilation artificielle selon la revendication 8, caractérisé par le fait qu'il comprend des moyens (27) de mesure du débit de fluide inspiré et/ou expiré par le patient placés juste en amont du patient.

11. Dispositif de ventilation artificielle selon la revendication 10, caractérisé par le fait que les dits moyens (11) de contrôle du volume courant mesurent pendant la phase d'inspiration et d'expiration et qu'il comprend des moyens de traitement du signal de débit ainsi mesuré pour d'une part détecter les instants de débit nul en fin d'inspiration et/ou d'expiration et d'autre part déterminer en fonction du débit mesuré le volume courant total VT inspiré par le patient.

12. Dispositif de ventilation artificielle selon la revendication 8, caractérisé par le fait qu'il comprend des moyens (12) d'incrémentation et/ou de décrémentation de la ventilation artificielle agissant sur l'alimentation en fluide sous pression (3) permettant d'arriver à ce que la relation "Vmn opt = FR x VTtot" soit satisfaite en fonction de la propre alimentation spontanée naturelle du patient en fluide gazeux respirable en respectant les dites consignes de référence.

13. Dispositif de ventilation artificielle selon la revendication 9, les dits moyens (6) de canalisation présentant au moins une canalisation d'inspiration (7), caractérisé par le fait que les dits moyens (13) comprennent :

    – une enceinte réservoir (14), dont le volume VC est déterminé, intercalée sur la dite canalisation d'inspiration (7) entre la dite alimentation sous pression et le patient, la dite enceinte (14) étant emplie en fluide gazeux sous pression à chaque cycle respiratoire à une pression de travail PT1 prédéterminée,

    – des moyens de décharge (16) du dit volume VTmach placés en aval de la dite enceinte (14), pour vider brutalement celle-ci et donner naissance à un pic (35) de débit du fluide au début de l'insufflation.

14. Dispositif de ventilation artificielle, la circulation du fluide gazeux dans le dit dispositif en phase d'inspiration et/ou d'expiration étant commandée par des électrovannes disposées au niveau des dits moyens de canalisation, selon la revendication 8, caractérisé par le fait qu'il comporte :

    – des moyens (28) de calcul et de commande substantiellement constitués par :

        – une unité centrale à microprocesseur permettant le déroulement séquentiel des différentes étapes,

        – une interface d'entrée (29, 30) recueillant les valeurs des consignes de référence telles que ventilation minute optimale, volume courant mini, pression maximale des voies aériennes, fréquence respiratoire maximale, et les informations mesurées telles que pression de travail dans l'enceinte, pression des voies aériennes, débit d'inspiration et/ou d'expiration.

        – une interface de sortie (31) contrôlant la commande séquentielle des électrovannes (15, 16, 19) pour autoriser, soit une ventilation spontanée, soit une ventilation contrôlée totale, soit une ventilation artificielle intermédiaire, et/ou la phase d'expiration.

## Patentansprüche

1. Verfahren zum Betrieb einer künstlichen Atmungsvorrichtung zur volumetrischen Einatmungshilfe eines Patienten (2), wobei der genannte Patient (2) eine eigene natürliche, spontane Atmung aufweist, die ein "VTpat" genanntes Patientenstromvolumen sowie einen Druck der Luftwege "Paw" bestimmt, wobei die genannte künstliche Atmungsvorrichtung (1) die Erzeugung eines "VTmach" genannten Maschinenstromvolumens und eine Reihe von Atmungszyklen je nach den Bedürfnissen des Patienten, mit einer Frequenz "FR", erlaubt, wobei jeder Atmungszyklus eine Einatmungsphase, während der dem Patienten ein Gesamtstromvolumen "VTtot" genanntes, atembares Volumen gasförmiges Fluidum zugeführt wird, das gleich der Summe des Patientenstromvolumens "VTpat", erhöht um das von der Maschine gelieferte Volumen "VTmach", d.h. "VTpat + VTmach", ist, sowie eine Ausatmungsphase aufweist, bei welchem Verfahren:

    – ein Referenz-Sollwert für die genannte Vorrichtung, der einem für den Patienten, je nach seinem Zustand und seiner natürlichen, spontanen Atmung zufriedigenden Atemvolumenwert entspricht, eingegeben wird, der optimales Minutenatemvolumen "Vmn.opt" genannt wird,

    – das dem Patienten bei jedem Atmungszyklus zugeführte Gesamtstromvolumen "VTtot" gesteuert wird, Verfahren dadurch gekennzeichnet, daß:

    – ein Referenz-Sollwert für die genannte Vorrichtung, der einem Niedrigstwert des Stromvolumens "VT mini" entspricht, mit "VTtot $\geq$ VT min", eingegeben wird,

    – ein Referenz-Sollwert für die genannte Vorrichtung, der einem für den Patienten Höchstwert des Drucks der Luftwege "Paw max" entspricht, eingegeben wird,

    – die künstliche Atmung abhängig vom Sollwert des Minutenatemvolumens "Vmn opt", des Niedrigst-Stromvolumens "VT mini" und des Höchstdrucks der Luftwege "Paw max" gesteuert wird, sodaß das Verhältnis "Vmn opt = FR x VT tot" entsprochen wird, um die künstliche Atmung automatisch vom spontanen Atmung zu einer gesteuerten Atmung, oder umgekehrt, je nach der natürlichen, spontanen Atmung des Patienten, evoluieren zu lassen.

2. Verfahren zum Betrieb einer künstlichen Atmungsvorrichtung nach Anspruch 1, dadurch gekennzeichnet, daß die künstliche Atmung, bei dem Einblasen während der Einatmungsphase, beim Anfang der Einatmung eine maximale Hilfe bei kleinem Atemvolumen liefert.

3. Verfahren zum Betrieb einer künstlichen Atmungsvorrichtung nach Anspruch 1, dadurch gekennzeichnet, daß das Gesamtstromvolumen "VTtot" während der Einatmungs- und Ausatmungsphase gesteuert und daß die Aufteilung des Zyklusses in Einatmungs- und Ausatmungsphasen gesteuert wird, wobei die Zeiten mit Nullflußmenge am Ende der Einatmung und am Ende der Ausatmung des Patienten festgestellt werden.

4. Verfahren zum Betrieb einer künstlichen Atmungsvorrichtung nach Anspruch 1, dadurch gekennzeichnet, daß der Wirkung der künstlichen Atmung, falls der Patient selbst dem Sollwert entspricht und das Verhältnis "Vmn opt = FR x VT tot" entspricht, aufgehoben oder sonst die künstliche Atmung bei jedem Zyklus erhöht wird, bis der Patient dem Sollwert mit der Einatmungshilfe entspricht.

5. Verfahren zum Betrieb einer künstlichen Atmungsvorrichtung nach Anspruch 4, dadurch gekennzeichnet, daß die künstliche Atmung, wenn der Sollwert mit der Einatmungshilfe entsprochen wird, allmählich vermindert wird, wobei die abhängige Steuerung solange behalten bleibt, bis der Patient aufneu seine natürliche, spontane Atmungsweise findet.

6. Verfahren zum Betrieb einer künstlichen Atmungsvorrichtung nach Anspruch 2, dadurch gekennzeichnet, daß dem Patienten bei jeder Einatmungsphase, wenn die künstliche Atmung in Betrieb ist, die Entladung eines bestimmten Volumens eines vorher auf einen voreingestellten Arbeitsdruck gebrachten, atembaren gasförmigen Fluidums eingeblasen wird.

7. Verfahren zum Betrieb einer künstlichen Atmungsvorrichtung nach Anspruch 1, dadurch gekennzeichnet, daß ein Höchstwert für die Atmungsfrequenz FR eingestellt wird, um eine Hyperatmungsgefahr zu vermeiden.

8. Künstliche Atmungsvorrichtung (1) zur Anwendung des Betriebsverfahrens nach Anspruch 1, wobei die genannte Vorrichtung umfaßt:

– eine Versorgungsquelle (3) für atembares gasförmiges Fluidum, die zumindest eine Fluidumversorgung unter Druck während der künstlichen Atmung erlaubt,

– Zufuhrmittel (6) für das genannte gasförmige Fluidum zum Patienten,

– Mittel (11) zur Steuerung des dem Patienten bei jedem Atmungszyklus zugeführten Gesamtstromvolumens "VTtot ",

– Mittel (10) zur Eingabe von Referenz-Sollwerten für die genannte Vorrichtung, die zimindest einem für den Patienten, je nach seinem Zustand und seiner natürlichen, spontanen Atmung zufriedigenden Atemvolumenwert entspricht, der optimales Minutenatemvolumen "Vmn.opt" genannt wird, Vorrichtung dadurch gekennzeichnet, daß:

– die genannten Mittel (10) zur Eingabe von Referenz-Sollwerten die Eingabe erlauben:

. eines Referenz-Sollwerts für die genannte Vorrichtung, der einem Niedrigstwert des Stromvolumens entspricht, das der Patient einatmen soll und Niedrigststromvolumen "VT mini" gennant ist, mit "VTtot $\geq$ VT mini",

. eines Referenz-Sollwerts für die genannte Vorrichtung, der einem Höchstwert des Drucks der Luftwege für den Patienten "Paw max" entspricht, und daß sie umfaßt:

– Mittel (12) zur Steuerung der künstlichen Atmung, die bei jedem Zyklus das Verhältnis "Vmn opt = FR x VTtot" nachprüfen und die Fluidumversorgung der künstlichen Atmung unter Druck je nach der spontanen, natürlichen Atmung des Patienten betätigt, um die künstliche Atmung automatisch zwischen der spontanen Atmung und einer gesteuerten Atmung evoluieren zu lassen.

9. Künstliche Atmungsvorrichtung nach Anspruch 8, dadurch gekennzeichnet, daß sie Mittel (13) umfaßt, um dem Patienten am Anfang der Einatmung eine maximale hilfe zu leisten, d.h. bei kleinem Atemvolumen, während der Einatmungsphase, bei der Einblasung über die genannten künstliche Atmungsvorrichtung.

10. Künstliche Atmungsvorrichtung nach Anspruch 8, dadurch gekennzeichnet, daß sie gerade stromaufwärts des Patienten angeordnete Mittel (27) zum Messen der Flußmenge des vom Patienten ein- und/oder ausgeatmeten Fluidums umfaßt.

11. Künstliche Atmungsvorrichtung nach Anspruch 10, dadurch gekennzeichnet, daß die genannten Mittel (11) zur Steuerung des Stromvolumens während der Ein- und Ausatmungsphase messen und daß sie Mittel zur Verarbeitung des also gemessenen Flußmengensignals umfaßt, um, einerseits, die Zeiten mit Nullflußmenge am Ende der Ein- und/oder Ausatmung festzustellen und, andererseits, je nach der gemessenen Flußmenge, den vom Patienten eingeatmeten Gesamtstromvolumen VT zu bestimmen.

12. Künstliche Atmungsvorrichtung nach Anspruch 8, dadurch gekennzeichnet, daß sie gerade stromaufwärts des Patienten angeordnete Mittel (12) zum Erhöhen und/oder Vermindern der künstlichen Atmung umfaßt, die die Zufuhr von Fluidum unter Druck (3) betätigen und es erlauben, zu erreichen, daß das Verhältnis "Vmn opt = FR x VTtot" je nach der eignen natürlichen, spontanen Zufuhr von atembarem gasförmigem Fluidum zum Patienten entsprochen wird, während die genannten Referenz-Sollwerte überwacht werden.

13. Künstliche Atmungsvorrichtung nach Anspruch 8, wobei die genannten Leitungsmittel (6) zumindest eine Einatmungsleitung (7) aufweisen, dadurch gekennzeichnet, daß die genannten Mittel (13) umfassen:

– einen auf der genannten Einsaugleitung (7) zwischen der genannten Zufuhr unter Druck und dem Patienten zwischengefügten Behälterraum (14), dessen Volumen VC bestimmt ist, wobei der genannte Raum bei jedem Atmungszuklus unter einem vorbestimmten Arbeitsdruck PT1 mit gasförmigen Fluidum gefüllt wird,

– stromabwärts des genannten Raums (114) angeordnete Ablaßmittel (16) für das genannte Volumen VTmach, um dieses letzte plötzlich abzulassen und eine Flußmengenspitze (35) des Fluidums am Anfang der Einblasung zu veranlassen.

14. Künstliche Atmungsvorrichtung, wobei der gasförmige Fluidumumlauf in der genannten Vorrichtung in der Ein- und/oder Ausatmungsphase über auf Höhe der genannten Leitungsmittel angeordnete Elektroventile gesteuert wird, nach Anspruch 8, dadurch gekennzeichnet, daß sie umfaßt:

– Rechnungs- und Steuermittel (28), im wesentlichen bestehend aus:

. einer Zentraleinheit mit Mikroprozeßor, die den Sequenzverlauf der verschiedenen Stufen erlaubt,

. eine Eingabeinterface (29, 30), die die Referenz-Sollwerte, wie optimales Minutenvolumen, Niedrigststromvolumen, Höchstdruck der Luftwege, Höchstatmungsfrequenz, und die Meßdaten, wie Arbeitsdruck im Behälterraum, Druck der Luftwege, Ein- und/oder Ausatmungsflußmenge, sammelt,

– eine Ausgabeinterface (31), die die Sequenzsteuerung der Elektroventile (15, 16, 19) überwacht, um entweder eine spontane Atmung oder eine vollgesteuerte Atmung oder eine künstliche Zwischenatmung und/oder die Ausatmungsphase zu erlauben.

## Claims

1. Process for operating an artificial ventilation device for providing volumetric inspiratory assistance to a patient (2), the said patient (2) having his own natural spontaneous ventilation defining a patient tidal volume, known as "VT pat", as well as an airway pressure "Paw", the said artificial ventilation device (1) permitting the generation of a machine tidal volume, known as "VTmach", and permitting a succession of respiratory cycles as a function of the patient's needs, having a frequency "FR", each respiratory cycle having an inspiration phase during which a volume of respirable gaseous fluid is supplied to the patient, this being known as the total tidal volume "VTtot" equal to the sum of the patient tidal volume "VTpat" plus the tidal volume delivered by the machine, "VTmach", namely "VTtot = VTpat + VTmach", as well as an expiratory phase, a process wherein:

– there is introduced a reference set value for the said device corresponding to a ventilation value satisfactory for the patient as a function of his condition and of his natural spontaneous ventilation, known as optimum ventilation per minute: "Vmn.opt",

– the total tidal volume delivered to the patient at each respiratory cycle: "VTtot" is monitored; the process being characterized by the fact that:

– a reference set value is introduced for the said device, this value corresponding to a minimum value of the tidal volume "VT mini", with "VTtot $\geqq$ VT mini",

– a reference set value is introduced for the said device, this value corresponding to a maximum pressure value of the airways for the patient: "Paw max",

– the artificial ventilation is slaved to the set value for ventilation per minute, with a minimum tidal volume "VT mini", and a maximum airway pressure "Paw max", in such a way as to satisfy the relation "Vmn opt = FR x VT tot" to cause the artificial ventilation to progress automatically from spontaneous ventilation to monitored ventilation, or conversely, as a function of the natural spontaneous ventilation of the patient.

2. Process for operating an artificial ventilation device according to claim 1, characterized by the fact that the artificial ventilation, at the time of insufflation during the inspiration phase provides maximum assistance at the commencement of inspiration when pulmonary volume is small.

3. Process for operating an artificial ventilation device according to claim 1, characterized by the fact that the total tidal volume "VTtot" is monitored during the inspiration and expiration phases and that the division of the cycle into inspiration and expiration phases is commanded by detecting the moments of zero flow at the end of inspiration and at the end of expiration by the patient.

4. Process for operating an artificial ventilation device according to claim 1, characterized by the fact that, if the patient himself conforms to the set value and satisfies the relation "Vmn opt = FR x VTtot", the artificial ventilation effect is eliminated, otherwise, the artificial ventilation is increased at each cycle up to the moment at which the patient conforms to the set value with inspiratory assistance.

5. Process for operating an artificial ventilation device according to claim 4, characterized by the fact that, when the set value is reached with inspiratory assistance, the artificial ventilation is progressively decremented while observing slaving up to the moment when the patient regains his natural spontaneous ventilation mode.

6. Process for operating an artificial ventilation device according to claim 2, characterized by the fact that, at each inspiration phase, when artificial ventilation is operative, there is insufflated into the patient the discharge of a given volume of respirable gaseous fluid previously brought to a pre-established working pressure.

7. Process for operating an artificial ventilation device according to claim 1, characterized by the fact that a maximum respiratory frequency value FR is fixed to forestall the risk of hyperventilation.

8. Artificial ventilation device (1) for implementing the operating process according to claim 1, the said device comprising:

– a source (3) for supplying respirable gaseous fluid, permitting at least a supply of fluid under pressure at the time of artificial ventilation,

– means (6) for channelling the said gaseous fluid to the patient,

– means (11) for monitoring the total tidal volume "VTtot" delivered to the patient at each respiratory cycle,

– means (10) for entering reference set values for the said device, corresponding at least to a ventilation value satisfactory for the patient as a function of his condition and of his natural spontaneous ventilation, known as optimum ventilation per minute: "Vmn.opt", the device being characterized by the fact that:

– the said means (10) for entering reference set values permit the entry of:

. a reference set value for the said device, corresponding to a minimum value of the tidal volume that the patient must breathe in, known as the minimum tidal volume: "VT mini", with "VTtot $\geqq$ VT mini",

. a reference set value for the said device, corresponding to a maximum airway pressure value for the patient: "Paw max", and that it comprises:

– means (12) for slaving the artificial ventilation verifying at each cycle the relation: "Vmn opt = FR x VT tot" acting on the supply of fluid under pressure at the time of artificial ventilation according to the spontaneous natural inspiration of the patient in order to cause the artificial ventilation to progress automatically between spontaneous ventilation and monitored ventilation.

9. Artificial ventilation device according to claim 8, characterized by the fact that it comprises means (13) for supplying maximum assistance to the patient at the commencement of inspiration, that is to say when the pulmonary volume is small, during the inspiration phase at the time of insufflation by the said artificial ventilation device.

10. Artificial ventilation device according to claim 8, characterized by the fact that it comprises means (27) for measuring the flow rate of fluid breathed in and/or breathed out by the patient placed just upstream of the patient.

11. Artificial ventilation device according to claim 10, characterized by the fact that the said means (11) for monitoring the tidal volume measure during the inspiration and expiration phases and that it comprises means for processing the flow rate signal thus measured for, one one hand, detecting the moments of zero flow at the end of inspiration and/or expiration and, on the other hand, determining, as a function of the flow rate measured, the total tidal volume VTtot breathed in by the patient.

12. Artificial ventilation device according to claim 8, characterized by the fact that it comprises means (12) for incrementing and/or decrementing the artificial ventilation acting on the pressurized fluid supply (3), making it possible to ensure that the relation "Vmn opt = FR x VTtot" is satisfied as a function of the patient's own natural spontaneous supply of respirable gaseous fluid while observing the said reference set values.

13. Artificial ventilation device according to claim 9, the said channelling means (6) having at least one inspiration channel (7), characterized by the fact that the said means (13) comprise;

– a reservoir (14), the volume VC of which is determined, intercalated on the said inspiration channel (7) between the said pressurized supply and the patient, the said reservoir (14) being filled with pressurized gaseous fluid at each respiratory cycle at a predetermined working pressure PT1,

– means (16) for discharging the said volume, VTmach, placed downstream of the said reservoir (14), for abruptly emptying the latter and giving rise to a peak (35) in the fluid flow rate at the commencement of insufflation.

14. Artificial ventilation device, the circulation of gaseous fluid in the said device in inspiration and/or expiration phase being commanded by solenoid valves disposed at the said channelling means, in accordance with claim 8, characterized by the fact that it comprises:

– computing and control means (28) substantially composed of:

– a microprocessor central unit permitting the sequential flow of the different stages,

– an input interface (29, 30) collecting the reference set values such as optimum ventilation per minute, minimum tidal volume, maximum airway pressure, maximum respiratory frequency, and the information measured, such as working pressure in the reservoir, airway pressure, inspiration and/or expiration flow rate,

– an output interface (31) monitoring the sequential control of the solenoid valves (15, 16, 19) to permit either spontaneous ventilation or total monitored ventilation, or again, intermediate artificial ventilation, and/or the expiration phase.

**FIG.1**

**FIG.2**

**FIG.3a**

**FIG.3b**

**FIG.3c**

VC PT1 —14

## FIG.4a

7 17

PB

VC PT2 —14

## FIG.4b

7 18

PT2

17

## FIG.5a

AIDE INSPIRATOIRE

SEQ

INITIALISATION DU SYSTEME EN VENTILA-SPONTANEE (PT1 = PB)

ENTREE DES CONSI-GNES VT, VMW, PAW, I/E, MODE

TTQ

SYSTEME ON

SEQ

CONTROLE DES CONSIGNES

AGORITHME DE REGULATION

AFFICHAGE DES RESULTATS

PRISE EN COMPTE DES NOUVELLES CONSIGNES

SEQ

MESURE DE PAW, VT, FR, I/E

SI

PRESSION PT2 < PAW MAX

PHASE 1 / FIG. 5B

PHASE 2 / FIG. 5C

FIG.5b

PHASE 1

SI

VOLUME COURANT
VT < CONSIGNE

AUGMENTATION DE LA
PRESSION PT1 DANS
L'ENCEINTE (14)

SI

VENTILATION
MINUTE
VMN < CONSIGNE

CONTROLE DU TEMPS
D'EXPIRATION RE-
PRISE INSUFFLATION

SI

PRESSION  PT1 >
PRESSION BAROMETRI-
QUE    PB

DIMINUTION DE LA
PRESSION PT1 DANS
L'ENCEINTE (14)

FIG.5c

PHASE 2

SEQ

TTQ

TTQ

PRESSION
PT2 > PAW MAX

TACHE 2.1
(REDUCTION DE-
BIT)/FIG. 5D

REDUCTION DEBIT
EXISTE

TACHE 2.2
/ FIG. 5C

## FIG.5d

```
                    ┌─────────────────┐
                    │   TACHE 2.1     │
                    └────────┬────────┘
                             │
┌──────────────┐         ╭───────╮         ┌──────────────┐
│ REDUCTION DU │─────────│  SEQ  │─────────│  TACHE 2.3   │
│ DEBIT        │         ╰───┬───╯         │   / FIG. 5F  │
└──────────────┘             │             └──────────────┘
                         ╭───────╮
                         │  SI   │
                         ╰───┬───╯
                  ┌──────────┴──────────┐
          ╱─────────────╲      ┌──────────────────┐
          │ VOLUME COURANT│     │ AUGMENTATION DE LA│
          │ VT < CONSIGNE │     │ PRESSION PT1      │
          ╲─────────────╱      │ DANS L'ENCEINTE   │
                                └──────────────────┘
```

## FIG.5e

```
                            ┌─────────────────┐
                            │   TACHE 2.2     │
                            └────────┬────────┘
                                     │
                                 ╭───────╮
          ┌──────────────────────│  SEQ  │──────────────────────┐
          │                      ╰───────╯                      │
      ╭───────╮                                          ┌──────────────┐
      │  TTQ  │──────────────┐                           │  TACHE 2.4   │
      ╰───┬───╯              │                           │   / FIG. 5I  │
  ╱─────────────╲            │                           └──────────────┘
  │ VOLUME COURANT│      ╭───────╮
  │ VT < CONSIGNE │──────│  SEQ  │──────────────────┐
  ╲─────────────╱      ╰───────╯                    │
        ┌──────────┬──────────────┐             ╭───────╮
  ┌─────────────┐ ┌──────────────┐  ┌───────────│  SI   │
  │AUGMENTATION DE│ │  TACHE 2.3   │             ╰───────╯
  │LA PRESSION PT1│ │   / FIG. 5F  │      ┌──────────┴──────────┐
  │DANS L'ENCEINTE│ └──────────────┘  ╱─────────────╲   ┌──────────────┐
  └─────────────┘                     │  PRESSION     │  │   PHASE 2    │
                                      │ PT2 > PAW MAX │  │   / FIG. 5C  │
                                      ╲─────────────╱   └──────────────┘
```

## FIG.5f

TACHE 2.3

SEQ

AFFICHAGE
DES
RESULTATS

PRISE EN
COMPTE DES
NOUVELLES
CONSIGNES

CONTROLE DES
CONSIGNES

MESURE DE PAW,
VT, VMN, I/E

SI

FREQUENCE DE
RESPIRATION
FR < CONSIGNE

CONTROLE DES TEMPS
T1 ET T2
D'INSUFFLATION
ET D'EXPIRATION

## FIG.5g

TACHE 2.4

SI

TEMPS T1 ET T2
CONTROLES

AUGMENTATION DU
DEBIT

SEQ

SUPPRESSION DU
CONTROLE DE T1, TE

MAINTIEN D'UNE AIDE
AU NIVEAU DE LA
CONSIGNE DE VT

TTQ

COMPTEUR < 5 MN

AFFICHAGE DES RE-
SULTATS FONTIONNE-
MENT EN CONTROLE

19